# EUROPEAN PATENT APPLICATION

(11) **EP 4 223 877 A1**
(43) Date of publication of application: **09.08.2023**
(21) Application number: 22155703.6
(22) Date of filing: 08.02.2022
(51) Int. Cl.: C12N 15/113, C12N 9/22

(54) **SYSTEM AND METHOD FOR EDITING GENOMIC DNA TO MODULATE SPLICING**

(71) Applicant: Eberhard Karls Universität Tübingen Medizinische Fakultät, 72074 Tübingen (DE)
(72) Inventor: Kohl, Susanne, 71154 Nufringen (DE); De Angeli, Pietro, 72076 Tübingen (DE); Wissinger, Bernd, 72127 Kusterdingen (DE)
(74) Representative: Witte, Weller & Partner Patentanwälte mbB

(57) **Abstract**

The invention is directed to a gene editing system for editing genomic DNA to modulate splicing, a polynucleotide or vector encoding said gene editing system, a lipid particle comprising said gene editing system, a pharmaceutical composition comprising said gene editing system polynucleotide or vector or lipid particle, methods of editing genomic DNA in a cell to modulate splicing, and a cell processed by said methods.

## Description

The invention is directed to a gene editing system for editing genomic DNA to modulate splicing, a polynucleotide or vector encoding said gene editing system, a lipid particle comprising said gene editing system, a pharmaceutical composition comprising said gene editing system polynucleotide or vector or lipid particle, methods of editing genomic DNA in a cell to modulate splicing, and a cell processed by said methods.

### FIELD OF THE INVENTION

The present invention relates to the field of molecular biology, more particular to the field of genetic engineering applications. It preferably relates to the field of to the targeted gene editing of genomic DNA.

### BACKGROUND OF THE INVENTION

RNA splicing is a process in molecular biology where a newly-made precursor messenger RNA, the so-called pre-mRNA transcript, is transformed into a mature messenger RNA (mRNA). It works by removing introns, i.e. the non-coding regions of RNA, and so joining together the exons, i.e. the coding regions. For nuclear-encoded genes, splicing occurs in the nucleus either during or immediately after transcription. For those eukaryotic genes that contain introns, splicing is usually needed to create an mRNA molecule that can be translated into protein. For many eukaryotic introns, splicing occurs in a series of reactions which are catalyzed by the spliceosome, a complex of small nuclear ribonucleoproteins (snRNPs). There exist self-splicing introns, that is, ribozymes that can catalyze their own excision from their parent RNA molecule.

Regulatory elements located within introns and exons guide the splicing complex, the spliceosome, and auxiliary RNA binding proteins to the correct sites for intron removal and exon joining. Misregulation of splicing and alternative splicing can result from mutations in cis regulatory elements within the affected gene, mutations *de novo* generating cis-regulatory elements, or from mutations that affect the activities of trans-acting factors that are components of the splicing machinery. Mutations that affect splicing can cause disease directly or contribute to the susceptibility or severity of disease.

It has been suggested that one third of all disease-causing mutations impact on splicing. Common errors include: (i) Mutation of a splice site resulting in loss of function of that site. Results in exposure of a premature stop codon, loss of an exon, or inclusion of an intron. (ii) Mutation of a splice site reducing specificity. May result in variation in the splice location, causing insertion or deletion of amino acids, or most likely, a disruption of the reading frame. (iii) Displacement of a splice site, leading to inclusion or exclusion of more RNA than expected, resulting in longer or shorter exons.

Inherited retinal dystrophies or diseases (IRD) are a heterogeneous group of genetic disorders with an estimated prevalence of 1 in 2,000 individuals. Over 250 genes are known to cause IRD and the inheritance can be autosomal dominant, autosomal recessive, X-linked recessive, and in rare instances non-Mendelian.

Retinitis pigmentosa (RP) is the most common sub form of IRD and characterized by night blindness and progressive degeneration of rod photoreceptors which leads to loss of peripheral vision, and eventually blindness in advanced stages. Dominant inherited RP (adRP) have been shown to account for more than 10% of all IRDs. Mutations in RHO encoding the apo-protein of the rod photoreceptor visual pigment is the most common cause of adRP. The Human Genome Mutation Database lists 247 likely disease-causing RHO variants, the vast majority (>200) being missense variants associated with adRP. Dominant RHO mutations typically act as gain-of-function or dominant-negative mutations and multiple biochemical disease mechanisms, such as protein misfolding and endoplasmatic reticulum (ER) retention, membrane trafficking deficiency, and dimerization defects have been described.

Stargardt disease is the most common single-gene IRD. It usually has an autosomal recessive inheritance caused by mutations in the *ABCA4* gene. Rarely it has an autosomal dominant inheritance due to defects with *PRPH2, ELOVL4* or *PROM1* genes. It is characterized by macular degeneration that begins in childhood, adolescence or adulthood, resulting in progressive loss of vision.

Usher syndrome is an autosomal recessively inherited rare ciliopathy characterized by sensorineural hearing loss and subsequent retinal degeneration (i.e. Retinitis pigmentosa, RP). Among the three clinical Usher subclasses, mutations in the *USH2A* gene are responsible for -50% of all Usher and 85% of Usher type 2 cases, respectively, and a common cause of isolated RP. Although many mutations in *USH2A* are private and therefore ultra rare, important founder mutations have been identified.

Numerous mutations associated with missplicing have been described as being associated with inherited retinal diseases; see, e.g., Qian et al. (2021), Identification of deep-intronic splice mutations in a large cohort of patients with inherited retinal diseases, Front. Genet. 12:647400. doi: 10.3389/fgene.2021.647400; Fadaie et al. (2021), Whole genome sequencing and in vitro splice assays reveal genetic causes for inherited retinal diseases, npj Genom. Med. 6, 97. doi.org/10.1038/s41525-021-00261-1.

Since the identification of the first gene responsible for an IRD back in 1988, enormous progress has been made in the field of molecular testing, leading to the identification of over 250 disease-causing genes. Nevertheless, until very recent times, these major diagnostic advances did not go hand in hand with the development of vision-sparing or vision-restoring therapeutic strategies and IRDs have been long accounted as largely incurable diseases. Over the last decades, novel therapeutic options started to be explored in preclinical studies, with some of them transitioning to the clinical setting, including gene therapy, cell therapy, retinal prosthetics, and even direct brain stimulation. In this context, gene-based therapies stand out as one of the most promising frontiers of IRD treatment; see Amato et al. (2021), Gene therapy in inherited retinal diseases: an update on current state of the art, Front. Med. doi.org/10.3389/fmed.2021.750586.

To date, the CRISPR/Cas system is considered the most advanced genome editing tool. Other than for strict genome editing, the CRISPR/Cas can also be used as part of a gene silencing strategy to inactivate mutant alleles causing a toxic gain-of-function, or as part of a splicing modulation approach to prevent the inclusion of pseudo-exons (i.e., deep-intronic sequences erroneously recognized as exons due to DNA mutations) that might result in the synthesis of an aberrant protein.

The CRISPR/Cas9-based transcript degradation approach has been used in studies on autosomal dominant retinitis pigmentosa (adRP) associated with rhodopsin mutations (RHO-adRP); see Tsai et al. (2018), Clustered regularly interspaced short palindromic repeats-based genome surgery for the treatment of autosomal dominant retinitis pigmentosa. Ophthalmology. 125:1421-30. doi: 10.1016/j.ophtha.2018.04.001; Li et al. (2018), Allele-specific CRISPR-Cas9 genome editing of the single-base P23H mutation for rhodopsin-associated dominant retinitis pigmentosa. CRISPR. (2018) 1:55-64. doi: 10.1089/crispr.2017.0009; Clement et al. (2019), CRISPResso2 provides accurate and rapid genome editing sequence analysis. Nat Biotechnol. 37:224-6. doi: 10.1038/s41587-019-0032-3; Bakondi et al. (2016), In Vivo CRISPR/Cas9 gene editing corrects retinal dystrophy in the S334ter-3 rat model of autosomal dominant retinitis pigmentosa. Mol Ther. 24:556-63. doi: 10.1038/mt.2015.220. The latter strategy of restoring splice defects has been applied to a deep intronic mutation of CEP290 in preclinical models; Garanto et al. (2013), Unexpected CEP290 mRNA splicing in a humanized knock-in mouse model for Leber congenital amaurosis. PLoS ONE. 8:e79369. doi: 10.1371/journal. pone.0079369.

Various CRISPR/Cas9-based gene editing systems have been examined in view of their mutational outcomes, including a system employing a Cas9-TREX2 fusion protein; see Allen et al. (2019), Predicting the mutations generated by repair of Cas9-induced double-strand breaks, Nature Biotechnology, Vol. 37, No. 1, 64-71.

The WO 2017/053879 proposes the use of exonucleases, such as TREX2, combined with an enzymatically active Cas9 nickase molecule, to improve genome editing.

The WO2018/009562 discloses CRISPR/Cas9-based compositions and methods for treating retinal degenerations.

In the WO2016/186772 methods for treating diseases associated with a deep intronic mutation using an engineered, non-naturally occurring CRISPR/Cas9-based system are disclosed.

However, so far no effective gene therapeutic approaches for modulating the splicing process have been made available in the art. Accordingly, there is an urgent need for such gene therapeutic strategies. In particular, such gene therapeutic systems are missing which allow a targeted rescue of missplicing events, but also allow the induction of missplicing events. However, both strategies could be useful to treat inherited diseases, in particular inherited retinal diseases. It is, therefore, an object underlying the invention to provide a novel gene editing system and methods of using it, by means of which the splicing process can be modulated in a targeted manner, thereby opening up new options for an effective treatment of genetic diseases, in particular inherited retinal diseases.

### SUMMARY OF THE INVENTION

This object is achieved by a gene editing system for editing genomic DNA to modulate splicing, comprising:
- a nucleic acid encoding an RNA-guided endonuclease molecule or fragment thereof fused to a molecule comprising exonuclease activity (ED-RgE molecule), and
- one single guide RNA (sgRNA) molecule,
wherein said ED-RgE molecule and said sgRNA molecule are configured to associate with genomic DNA comprising a splicing site and to cause a deletion in said splicing site resulting in a modulation of splicing.

This object is also achieved by a gene editing system for editing genomic DNA to modulate splicing comprising:
- a fusion protein comprising an RNA-guided endonuclease molecule or fragment thereof fused to a molecule comprising exonuclease activity (ED-RgE molecule), and
- one single guide RNA (sgRNA molecule),
wherein said ED-RgE molecule and said sgRNA molecule are configured to associate with genomic DNA comprising a splicing site and to cause a deletion in said splicing site resulting in a modulation of splicing.

The object is likewise achieved by the use of a nucleic acid encoding an RNA-guided endonuclease molecule or fragment thereof fused to a molecule comprising exonuclease activity (ED-RgE molecule) for the modulation of splicing, and by the use of the ED-RgE molecule for the modulation of splicing.

According to the invention a "nucleic acid" includes any kind of biopolymers composed of nucleotides, such as deoxyribonucleic acid (DNA) and ribonucleic acid (RNA).

According to the invention "gene editing" generally refers to genetic engineering where nucleic acid, in particular deoxyribonucleic acid (DNA), is modified, e.g. by nucleotide deletion, insertion and/or replacement etc. in a target specific manner.

According to the invention "splicing", also referred to RNA splicing, mean a process naturally occurring in eukaryotic cells, where a newly-made precursor messenger RNA (pre-mRNA) transcript is transformed into a mature messenger RNA (mRNA). It works by removing introns (non-coding regions of RNA) and so joining together exons (coding regions). For nuclear-encoded genes, splicing occurs in the nucleus either during or immediately after transcription. For those eukaryotic genes that contain introns, splicing is usually needed to create an mRNA molecule that can be translated into protein.

According to the invention a "modulation of splicing" means a targeted and specific molecular influence on the splicing process. In an embodiment such modulation includes a splicing rescue, i.e. a prevention or repair of incorrect splicing or missplicing by ensuring a processing of pre-mRNA into correct mRNA. Such rescue can, in an embodiment, realized by removing false exons or so-called pseudoexons. In an alternative embodiment of the invention such modulation includes an induction of missplicing, i.e. an influencing of the splicing process to effect a processing of pre-mRNA into incorrect mRNA, e.g. by inducing the "skipping" of a canonical exon.

According to the invention an "RNA-guided endonuclease" refers to an enzyme which is directed to a polynucleotide chain by a specific RNA molecule or a guide RNA, where it cleaves phosphodiester bonds. Included are single-strand and double-strand endonucleases. Suitable RNA-guide exonucleases include, without being restricted thereto, CRISPR associated protein 9, 12, 3 (Cas9, Cas12, Cas3) etc.

A "fragment" of an RNA-guided endonuclease refers to a part or section or a domain of the entire enzyme having RNA-guided exonuclease activity. I.e., a fragment can differ from the entire enzyme, e.g., by at least one or more amino acid residues, but still retains functional exonuclease activity. Functional activity of a domain or fragment of a naturally occurring RNA-guided exonuclease described herein can be tested using functional assays for exonuclease activity known in the art. Using a fragment instead of an entire full-length enzyme can result in a higher expression level, increased enzymatic activity, simplifications in the formulation into a pharmaceutical composition, and finally lower production efforts and costs.

According to the invention a "molecule comprising exonuclease activity" refers to a peptide, polypeptide or protein acting by enzymatically cleaving nucleotides enzymatic one at a time from the end (exo) of a polynucleotide chain. It includes any kind of exonuclease that can be fused to the RNA-guided endonuclease, e.g., in an preferred embodiment the so-called three prime repair exonuclease 2 (TREX2).

Specific endonucleases, so-called meganucleases, fused to exonucleases are generally well-known in the art, e.g., from Delacôte et al. (2013), High frequency targeted mutagenesis using engineered endonucleases and DNA-end processing eEnzymes. PLoS ONE 8(1): e53217.

The inventors refer to the fusion molecule consisting of RNA-guided endonuclease molecule and the molecule comprising exonuclease activity as 'enhanced deletion RNA-guided endonuclease molecule', abbreviated as "ED-RgE", because it is able to promote larger deletions in the DNA compared to the RNA-guided endonuclease without the fused exonuclease.

According to the invention a "single guide RNA (sgRNA)" refers to a piece of RNA that functions as a guide for the RNA-guided endonuclease, which it forms a complex with. The sgRNA is designed to specifically bind to or in close vicinity to a splicing site on the genomic DNA, thereby guiding the ED-RgE molecule to the latter. The design involves the incorporation of nucleotides resulting in a sequence which is complementary or at least essentially complementary to the nucleotide sequence of the splicing site or a sequence in close vicinity thereto, or a section thereof. A typical sgRNA has a length of approx. 17-24bp but can be longer or shorter. In an embodiment of the invention where Cas9 is used as the RNA-guided endonuclease the desired target sequence of the splicing site must precede the so-called protospacer adjacent motif (PAM) which is a short DNA sequence usually 2-6 base pairs in length that follows the DNA region targeted for cleavage by the gene editing system.

According to the invention the gene editing system comprises only one sgRNA molecule whereas in the art mostly two sgRNA molecules are used. "One" sgRNA is meant to refer to only one type of sgRNA defined by a particular nucleotide sequence. This means that the gene editing system according to the invention when used in solution consists of only one population of sgRNA molecules defined by a uniform or identical nucleotide sequence. Using one sgRNA compared to two sgRNAs or gRNAs as typically used in the art, has the following advantages: 1. Using a single/one sgRNA does not have the potential of generating structural aberrations, which is the case for two sgRNAs. 2. The single/one sgRNA can be designed to span a given mutation resulting in only targeting of the specific mutation allele, leaving the other allele intact. 3. Using a single/one sgRNA to target only the mutant allele reduces the chance for recombination between the homologous alleles generating an undesired outcome. 4. Using a single/one sgRNA reduces the change of p53 activation in cells due to the generation of multiple doublestranded breaks. 5. Using a single/one sgRNA reduces the chances of off-target mutagenesis. 6. Using a single/one sgRNA enables allele-specific genome editing.

According to the invention a "splicing site" refers to any sequence of nucleotides on the genomic DNA which is involved in or acting on the splicing process. A splicing site, according to the invention, includes intronic variants/mutations, deep intronic variants/mutations, single nucleotide polymorphisms (SNP), intronic and exonic SNPs, canonical splice sites or sequences, donor splice sites, acceptor splice sites, branch points, slicing enhancers, splicing silencers, etc. Typically, a splicing site according to the invention may have a length of approx. 1 nt. - 50 nt.

The inventors have realized that the gene editing system according to the invention generates deletions on the genomic DNA, which are larger than the ones generated by non-fused, "normal" RNA-guided endonucleases. It also enhances the frequency of occurrence of deletions in comparison to non-fused, "normal" RNA-guided endonucleases. These effects lead to the disruption of the splicing sequence. Upon the generation of a double-strand break mediated by the RNA-guided endonuclease paired to the sgRNA, the fused exonuclease is able to further process the generated ends, leading to larger deletions. This translates, in comparison to non-fused, "normal" RNA-guided endonuclease, in a splicing modulation with a higher degree or percentage. Moreover, the ED-RgE according to the invention enables flexibility in sgRNA design as its disruption activity is also seen at a distance from the splicing site.

The problem underlying the invention is herewith fully solved.

In an embodiment of the gene editing system of the invention said RNA-guided endonuclease molecule is a Cas9 molecule.

This measure has the advantage that such an RNA-guided endonuclease is used which is particularly suitable for the purposes of the invention. Cas9 (CRISPR associated protein 9, formerly called Cas5, Csn1, or Csx12) is a 160 kilodalton protein which has been proven to induce site-directed strand breaks in DNA in many genetic engineering applications. Cas9 is a dual RNA-guided DNA endonuclease enzyme associated with the Clustered Regularly Interspaced Short Palindromic Repeats (CRISPR) system. Cas9 can cleave nearly any sequence complementary to the sgRNA. Because the target specificity of Cas9 stems from the guide RNA:DNA complementarity and not from modifications to the protein itself (like TALENs and zinc fingers), engineering Cas9 to target new DNA is straightforward. The induction of DNA strand breaks by Cas9 can lead to the inactivation of the splicing site or the introduction of heterologous sequences through non-homologous end joining and homologous recombination, respectively.

In another embodiment of the invention said Cas9 is selected from the group consisting of: *Streptococcus pyogenes* Cas9 (SpCas9), Neisseria meningitides Cas9 (Nme2Cas9), and *Staphylococcus auricularis* Cas9 (SauriCas9).

The measure has the advantage that such Cas9 enzymes are used which achieve particularly good results in the invention. While the use of standard SpCas9 has been demonstrated in numerous applications to ensure a solid editing activity Nme2Cas9 and SauriCas9 have the advantage of being small Cas9 orthologous which are active in mammalian cells and which can be packed in AAV particles; see Edraki et al. (2019), A compact, high-accuracy Cas9 with a dinucleotide PAM for in vivo genome editing. Mol Cell 73: 714-726; Hu et al. (2020), A compact Cas9 ortholog from Staphylococcus auricularis (SauriCas9) expands the DNA targeting scope. PLoS Biol 18: e3000686. Moreover, due to the permissive protospacer adjacent motif (PAM) requirements (N4CC for Nme2Cas9, NNGG for SauriCas9 and NGG for SpCas9) the availability of various Cas9 orthologues results in a higher flexibility in targeting defined DNA sequences.

In another embodiment of the gene editing system of the invention said one sgRNA molecule is configured to direct said ED-RgE molecule to said splicing site.

This function is achieved in an advantageous manner by using an sgRNA molecule comprising a nucleotide sequence which is complementary or essentially complementary to the nucleotide sequence of the splicing site.

In an embodiment of the editing system of the invention said modulation of splicing is a splicing rescue.

A "splicing rescue" or synonymously "splicing correction" refers to a mode of action resulting in a correct splicing process and, finally, a correctly matured mRNA. According to the invention this result can be achieved by numerous mechanisms that occur after being triggered by the deletion in the splicing site. Such mechanisms include, e.g., the removal of an incorrect splicing signal, an intronic sequence or a pseudoexon, or the entire splicing site, etc. In this embodiment, the gene editing system is adapted to a use in an advantageous manner where the genomic DNA comprises splicing signals which finally result in a misspliced mRNA. For example, the incorrect splicing signal may be or result from a deep-intronic variant which finally could manifest in the inclusion of an intronic sequence or a pseudoexon, respectively, during the splicing process. The inclusion of the intronic sequence can happen because there might be intronic sequences closely resembling splicing signals and positioned in a way similar to normal exons.

In an alternative embodiment of the invention said modulation of splicing is a missplicing.

In this embodiment missplicing or synonymously aberrant splicing can be induced purposely by the gene editing system according to the invention. This can be achieved by numerous mechanisms that occur after being triggered by the deletion in the splicing site. E.g. a skipping of a canonical exon can be induced resulting in a missplicing and the failure of protein production. In this embodiment, the gene editing system is adapted to a use where mutations can result in a dominant acting-effect, as it is observed in the *RHO* gene. There, one mutated allele is enough to determine the onset of a disease. In this embodiment, e.g., common single nucleotide polymorphisms (SNPs) that are in heterozygosity (representing "splicing sites" as defined above) are targeted or, more, specifically, a nucleotide that is on the same allele as the mutation is targeted.

In yet another embodiment of the invention said splicing site is selected from the group consisting of: intronic variant/mutation, deep intronic variant/mutation, single nucleotide polymorphism (SNP), intronic and exonic SNP, donor splice site, acceptor splice site, branch point, splicing enhancer, splicing silencer.

This measure has the advantage that such a splicing site is addressed or targeted by the gene editing system of the invention which allows an effective modulation of the splicing process.

In still another embodiment of the invention said molecule comprising exonuclease activity comprises TREX2 exonuclease activity.

By this measure such an exonuclease is used which, according to the findings of the inventors, results in a very high splicing modulation activity of the gene editing system of the invention. TREX2 (Three prime repair exonuclease 2) is an enzyme that in humans is encoded by the *TREX2* gene (Human: Entrez 11219; Ensembl: ENSG00000183479; UniProt: Q9BQ50; RefSeq (mRNA) NM_080701, NM_080699, NM_080700; RefSeq (protein) NP_542432). The protein has 3' exonuclease activity. "Comprising TREX2 exonuclease activity" includes a molecule which is TREX2 exonuclease.

In another embodiment of the gene editing system according to the invention said ED-RgE molecule comprises a human TREX2 exonuclease fused to a SpCas9 molecule resulting in an "ED-Cas9 molecule". In still another embodiment of the invention said ED-Cas9 molecule comprises a nucleotide sequence which is at least 85% identical to the nucleotide sequence of SEQ ID NO: 58, and/or a nucleotide sequence which is at least 85% identical to the nucleotide sequence of SEQ ID NO: 60. In another embodiment of the invention said ED-Cas9 molecule comprises an amino acid sequence which is at least 85% identical to the amino acid sequence of SEQ ID NO: 59, and/or an amino acid sequence which is at least 85% identical to the amino acid sequence of SEQ ID NO: 61.

By this measure such an ED-RgE or ED-Cas9 molecule is provided which is particularly effective in the modulation of the splicing process. A variant sequence which is at least 85% identical refers to a sequence, having, over its entire length, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or about 100% sequence identity with the entire length of reference nucleotide or amino acid sequences which encode full-length SpCas9 or TREX2, respectively. The inventors have realized that also such variants of SpCas9 or TREX2 can be used, which differ in the encoding nucleotide sequences or amino acid sequences from the full-length counterparts but retain the respective enzymatic activities.

In the present invention, the term "identical" (synonym "homologous") refers to the degree of identity between sequences of two nucleotide or amino acid sequences. The aforementioned "identity" is determined by comparing two sequences aligned under optimal conditions over the sequences to be compared. Such a sequence homology can be calculated by creating an alignment using, for example, the ClustalW algorithm. Commonly available sequence analysis software, more specifically, Vector NTI, GENETYX or other tools are provided by public databases.

"Percent identity" or "percent identical" in turn, when referring to a sequence, means that a sequence is compared to a claimed or described sequence after alignment of the sequence to be compared (the "Compared Sequence") with the described or claimed sequence (the "Reference Sequence"). The percent identity is then determined according to the following formula: percent identity = 100 [1 -(C/R)]
wherein C is the number of differences between the Reference Sequence and the Compared Sequence over the length of alignment between the Reference Sequence and the Compared Sequence, wherein
(i) each nucleotide or amino acid in the Reference Sequence that does not have a corresponding aligned nucleotide or amino acid in the Compared Sequence and
(ii) each gap in the Reference Sequence and
(iii) each aligned nucleotide or amino acid in the Reference Sequence that is different from an aligned nucleotide or amino acid in the Compared Sequence, constitutes a difference and
(iv) the alignment has to start at position 1 of the aligned sequences;
   and R is the number of nucleotides or amino acids in the Reference Sequence over the length of the alignment with the Compared Sequence with any gap created in the Reference Sequence also being counted as a nucleotide or amino acid.

If an alignment exists between the Compared Sequence and the Reference Sequence for which the percent identity as calculated above is about equal to or greater than a specified minimum Percent Identity then the Compared Sequence has the specified minimum percent identity to the Reference Sequence even though alignments may exist in which the herein above calculated percent identity is less than the specified percent identity.

Methods for comparing the identity/homology of two or more sequences are known in the art. For example, the "needle" program, which uses the Needleman-Wunsch global alignment algorithm (Needleman and Wunsch, 1970 J. Mol. Biol. 48:443-453) to find the optimum alignment (including gaps) of two sequences when considering their entire length may be used. The needle program is for example available on 30 the World Wide Web site and is further described in the following publication (EMBOSS: The European Molecular Biology Open Software Suite (2000) Rice, P. Longden, I. and Bleasby, A. Trends in Genetics 16, (6) pp. 276-277). The percentage of identity between two polypeptides, in accordance with the disclosure, is calculated using the EMBOSS: needle (global) program with a "Gap Open" parameter equal to 10.0, a "Gap Extend" parameter equal 35 to 0.5, and a Blosum62 matrix.

In an embodiment of the invention said genomic DNA encodes a gene associated with an inherited disorder, preferably a retinal disease, further preferably an inherited retinal dystrophy (IRD).

By this measure the invention is adapted for a use in the treatment of any inherited disorder, in particular specific retinal disorders where the gene editing system of the invention promises particularly good results.

In another embodiment of the invention said gene comprised by the genomic DNA is selected from the group consisting of: *ABCA4, RHO, USH2A, GUCY2D, RP1L1,* and *PROM1* preferably of human origin.

By this measure the invention is configured for an editing of such genes which are known of being involved in inherited retinal disorders and which are especially suitable for a treatment by the gene editing system of the invention.

In yet another embodiment of the invention said splicing site is selected from the group consisting of:
- *ABCA4:* c.5197-557G>T, c.5196+1013A>G, c.5196+1056A>G, c.5196+1134C>G, c.5196+1137G>A, c.5196+1216 C>A; and/or
- *RHO*: c.360C>T, c.696+4C>T, c.68C>A, c.937-23G>A, c.360C>T; and/or
- *USH2A:* c.7595-2144A>G.

The inventors have realized that the afore-mentioned sites can be effectively addressed by the editing system of the invention as they are involved in the splicing processes of the underlying genes recited in italic letters.

*ABCA4* (ATP-binding cassette, sub-family A (ABC1), member 4) encodes for a member of the ATP-binding cassette transporter gene sub-family A (ABC1) found exclusively in multicellular eukaryotes. The ABCA4 protein is almost exclusively expressed in retina localizing in outer segment disk edges of rod photoreceptors. Mutations in *ABCA4* gene are known to cause the autosomal-recessive disease Stargardt disease or Stargardt macular dystrophy (STGD1), which is a hereditary juvenile macular degeneration disease causing progressive loss of photoreceptor cells, but also *fundus flavimaculatus,* cone- and cone-rod dystrophy and *retinitis pigmentosa.* Human: Entrez 24; Ensembl ENSG00000198691; UniProt P78363; RefSeq (mRNA) NM_000350; RefSeq (protein) NP_000341.

*RHO* (rhodopsin) encodes for a light-sensitive receptor protein involved in visual phototransduction. Rhodopsin is a biological pigment found in the rods of the retina and is a G-protein-coupled receptor (GPCR). It belongs to a group of photoswitchable opsins. Mutation of the *RHO* gene is a major contributor to various retinopathies such as *retinitis pigmentosa.* Human: Entrez 6010; Ensembl ENSG00000163914; UniProt P08100; RefSeq (mRNA) NM_000539; RefSeq (protein) NP_000530.

USH2A (Usher syndrome 2A) encodes the protein Usherin that contains laminin EGF motifs, a pentraxin domain, and many fibronectin type III motifs. The encoded basement membrane-associated protein may be important in development and homeostasis of the inner ear and retina. Mutations within this gene have been associated with Usher syndrome type IIa, i.e. hearing loss and *retinitis pigmentosa,* and isolated *retinitis pigmentosa.* Alternatively, spliced transcript variants that encode different isoforms have been described. Human: Entrez 7399; Ensembl ENSG00000042781; UniProt O75445; RefSeq (mRNA) NM_206933, NM_007123; RefSeq (protein) NP_009054, NP_996816.

Another subject-matter of the invention is a polynucleotide or vector encoding the gene editing system according to the invention.

A "polynucleotide" is a biopolymer composed of 13 or more nucleotide monomers covalently bonded in a chain. DNA (deoxyribonucleic acid) and RNA (ribonucleic acid) are examples of polynucleotides. According to the invention, a "vector" includes any DNA molecule used as a vehicle to artificially carry a nucleic acid molecule, such as a nucleic acid encoding the ED-RgE molecule and/or the sgRNA into another cell, where it can be replicated and/or expressed. Viral vectors are of particular preference as they are characterized by embodying the viruses' specialized molecular mechanisms to efficiently transport genomes inside the cells they infect. The vector may or may not be, in a preferred embodiment of the invention, an adeno-associated vector (AAV), a lentivirus vector, etc. Further suitable vectors include xCas9-exo backbone vectors. In an embodiment the vector may comprise nuclear localization sequences (NLS), e.g., added to the N- and/or C-termini of molecule comprising exonuclease activity, such as TREX2, and/or the RNA-guided endonuclease molecule or fragment thereof, respectively. The vector according to the invention may also comprise a detectable marker, e.g. T2A-EGFP marker. A preferred vector is described in the examples' part.

The features, characteristics, advantages and embodiments described for the gene editing system according to the invention also apply to the polynucleotide and vector according to the invention.

Another subject-matter of the invention relates to a lipid particle comprising or carrying the gene editing system and/or the polynucleotide and/or vector according to the invention.

The features, characteristics, advantages and embodiments described for the gene editing system according to the invention also apply to the lipid particle according to the invention.

A still further subject-matter of the invention relates to a pharmaceutical composition comprising the gene editing system and/or the polynucleotide and/or vector and/or lipid particle according to the invention and a pharmaceutically acceptable carrier.

A "pharmaceutical composition" is a composition suitable for an administration to an animal and/or human being in a medical setting. Preferably, a pharmaceutical composition is sterile and produced according to GMP guidelines.

'Pharmaceutically acceptable carriers' or excipients are well known in the art and include, for example, nanocarriers, nanovectors, aqueous solutions such as water or physiologically buffered saline or other solvents or vehicles such as glycols, glycerol, oils such as olive oil or injectable organic esters. In preferred embodiments, when such pharmaceutical compositions are for human administration, e.g., for parenteral administration, the aqueous solution is pyrogen-free, or substantially pyrogen-free. The excipients can be chosen, for example, to effect delayed release of an agent or to selectively target one or more cells, tissues or organs. The pharmaceutical composition can be in dosage unit form such as an injection, tablet, capsule (including sprinkle capsule and gelatin capsule), granule, powder, syrup, suppository, or the like. The composition can also be present in a solution suitable for topical administration. Alternatively, the pharmaceutical composition can be present in form of an aerosol administrable by inhalation.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. Suitable pharmaceutical carriers or excipients as well as pharmaceutical necessities for use in pharmaceutical formulations are described in Remington - The Science and Practice of Pharmacy, 23rd edition, 2020, a well-known reference text in this field, and in the USP/NF (United States Pharmacopeia and the National Formulary). Other sources are also available to one of ordinary skill in the art.

The pharmaceutical composition as well as the methods to be explained below of the present invention may be utilized to treat a living being in need thereof. In certain embodiments, the living being is a mammal such as a human, or another non-human mammal.

The features, characteristics, advantages and embodiments described for the gene editing system according to the invention also apply to the pharmaceutical composition according to the invention.

Another subject-matter of the invention is a method of editing genomic DNA in a cell to modulate splicing, the method comprising
- contacting the cell with a
   - a nucleic acid encoding an RNA-guided endonuclease molecule or fragment thereof fused to a molecule comprising exonuclease activity (ED-RgE molecule), and
   - one single guide RNA (sgRNA molecule), wherein said ED-RgE molecule and said sgRNA molecule are configured to associate with genomic DNA comprising a splicing site and to cause a deletion in said splicing site resulting in a modulation of splicing,
- incubating the cell under conditions allowing
   - said ED-RgE molecule to by synthesized;
   - said ED-RgE molecule and said sgRNA molecule to associate with said genomic DNA, and
   - to cause a deletion in said splicing site resulting in the modulation of splicing.

Yet another subject-matter relates to a method of editing genomic DNA in a cell to modulate splicing, the method comprising
- contacting the cell with a
   - a fusion protein comprising an RNA-guided endonuclease molecule or fragment thereof fused to a molecule comprising exonuclease activity (ED-RgE molecule), and
   - one single guide RNA (sgRNA molecule),
      wherein said ED-RgE molecule and said sgRNA molecule are configured to associate with genomic DNA comprising a splicing site and to cause a deletion in said splicing site resulting in a modulation of splicing,
- incubating the cell under conditions allowing
   - said ED-RgE molecule and said sgRNA molecule to associate with said genomic DNA, and
   - to cause a deletion in said splicing site resulting in the modulation of splicing.

In an embodiment of the invention said methods are carried-out *in vitro.* The cell might be a prokaryotic or a eukaryotic cell, such as an animal cell, a vertebrate cell, a mammalian cell or a human cell. In an alternative embodiment the method can be carried out *in vivo,* e.g. the 'contacting the cell with' can be understood as or, instead, replaced by 'introducing into a living being'.

The features, characteristics, advantages and embodiments described for the gene editing system according to the invention also apply to the afore-referenced methods according to the invention.

Another subject-matter of the present invention relates to a cell processed by the methods according to the invention, and a pharmaceutical composition comprising said cell.

The cell might be a prokaryotic or a eukaryotic cell, such as an animal cell, a vertebrate cell, a mammalian cell or a human cell.

The features, characteristics, advantages and embodiments described for the gene editing system according to the invention also apply to the afore-referenced cell according to the invention.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

### BRIEF DESCRIPTION OF THE FIGURES

- Fig. 1:: Explicative example of the pathological mechanism determined by most deep-intronic variants.
- Fig. 2:: Explicative example on how to target DIVs with a sgRNA complexed with *Streptococcus pyogenes* Cas9 (SpCas9).
- Fig. 3:: Explicative example on how to target DIVs with a sgRNA complexed with the bioengineered ED-Cas9 variant.
- Fig. 4:: Vector map of ED-Cas9.
- Fig. 5: Minigene assay results in HEK293T cells for *ABCA4* c.5197-557G>T, ED-Cas9 compared to SpCas9 (n=3).
- Fig. 6:: mRNA rescue of several ABCA4 deep-intronic variants in intron 36.
- Fig. 7:: Details of the pseudo-exons determined by ABCA4 c.5196+1013A>G and ABCA4 c.5196+1056A>G (top) and ABCA4 c.5196+1134C>G and ABCA4 c.5196+1216 C>A (bottom).
- Fig. 8:: Mutational profiling of SpCas9 and ED-Cas9 coupled to gRNA25 and gRNA26 in patient-derived photoreceptor precursor cells.
- Fig. 9: Scheme of the molecular principle of the SEDIMS-X approach adapted to five common SNPs in *RHO*. Mutant alleles are specifically cleaved by gRNAs targeting SNPs (red crosses) in *cis* with the principle mutation and efficiently destructed by the exonuclease activity (red arrows) of the Cas9-exo fusion.
- Fig. 10: Pilot experiment comparing the gene disruption efficacy of SpCas9-exo and basic SpCas9 targeting c.694+4 in *RHO*. HEK293 cells were co-transfected with pSPL3-RHO and SpCas9-exo/U6.gRNA or SpCas9/U6gRNA (two alternative gRNAs, gRNA1 and gRNA2, were used) and minigene derived transcripts analyzed by RT-PCR and Sanger sequencing. The SpCas9-exo variant more efficiently induce missplicing (upper band), e.g. disruption of the exon 3 - intron 3 boundary and retention of 109 nt. intronic sequence resulting in a frameshift and a premature stop.

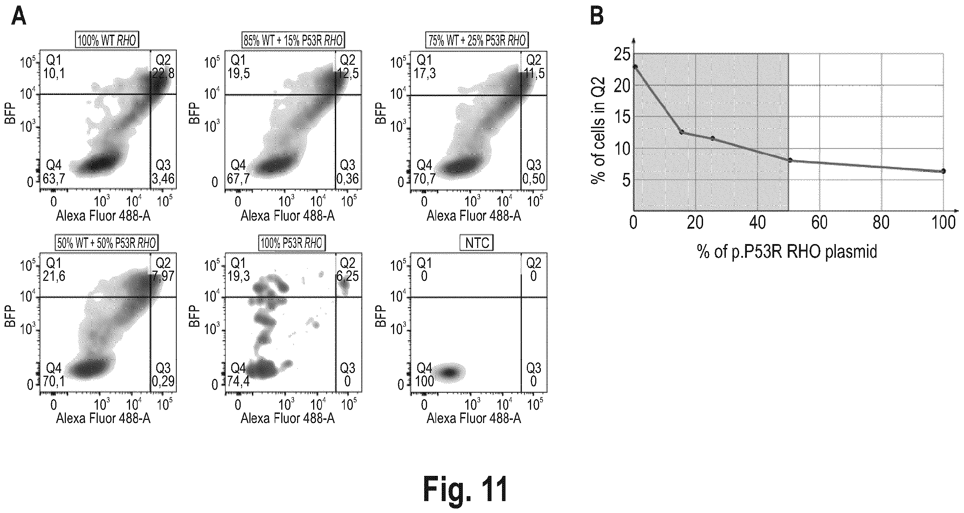

- Fig. 11:: (A) Localization of RHO p.Pro53Arg mutant proteins on the plasma membrane determined by flow cytometry. HEK293T cells were co-transfected with different ratios of mutant RHO plasmids and wild-type (WT) plasmid. BFP is used as marker for detecting transfected cells whereas Alexa Flour 488-A detects the RHO protein localized on the plasma membrane. The Q2 quadrant refers to cells containing the RHO plasmids and highly expressing the RHO protein on the membrane surface. NTC= Non-Transfected Control (B) % of cells in Q2 plotted versus % of P53 RHO plasmid used in the transfection. The shaded area indicates the range for rescue by selective disruption of the mutant allele in heterozygotes.
- Fig. 12:: (A) *USH2A*:c.7595-2144A>G induces the retention of a 152-bp pseudoexon (PE40) in the mature mRNA transcript. The presence of premature stop codons in the aberrant mRNA activates the degradation of these transcript via nonsense mediated decay (NMD). No protein translation occurs and no USH2A protein is produced. (B) Single gRNA-EDSpCas9 edits intronic sequences essential for PE40 recognition, hereby restoring splicing back to normal, and functional USH2A protein can again be produced.
- Fig. 13: Left: 30-day differentiated PPCs stained for the ciliary marker ARL13B (red, white arrow heads; blue - DAPI). Right: RT-PCR for *USH2A* expression (exon 2 to exon 3 amplified) in iPSCs and PPCs (250ng cDNA used as template). NC= negative control.

### EXAMPLES

### 1. Molecular mechanism/principle of the ED-Cas9 approach applied to deep-intronic variants

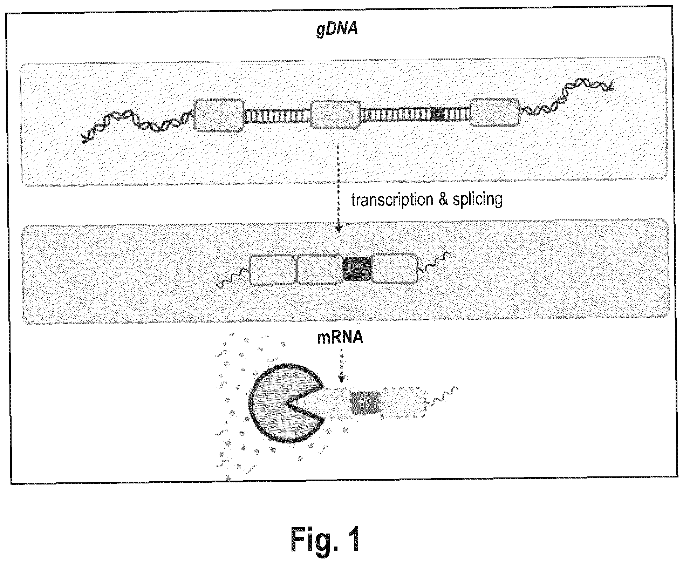

An explicative example of the pathological mechanism determined by most deep-intronic variants is depicted in Fig. 1. The presence of deep intronic variants (DIVs) resulting in missplicing by the retention of an intronic sequence (=pseudoexon, PE) in the mature transcript often lead to premature stop codon formation, and thus degradation of the mutant transcript by nonsense mediated mRNA decay. The DIV itself "activates" a cryptic splice site that is recognized as an alternative splicing signal, determining missplicing.

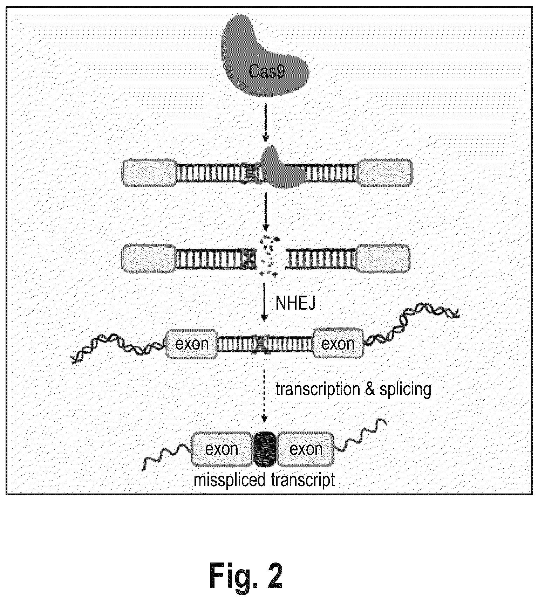

In Fig. 2 an explicative example is shown on how to target DIVs with a sgRNA complexed with *Streptococcus pyogenes* Cas9 (SpCas9). In principle, the deletion of a DIV can lead to the rescue of the correctly spliced form of the gene transcript. When targeting the DIV by a sgRNA paired to the wild-type *Streptococcus pyogenes* Cas9 (SpCas9) that can cut near the DIV itself, this typically generates a double-strand break, and the mutational profiles generated by the cell repair mechanism are mainly small deletions or/and insertions. Part of these repair profiles are often not able to perturb the strength of the cryptic splice site, hereby not rescuing the missplicing.

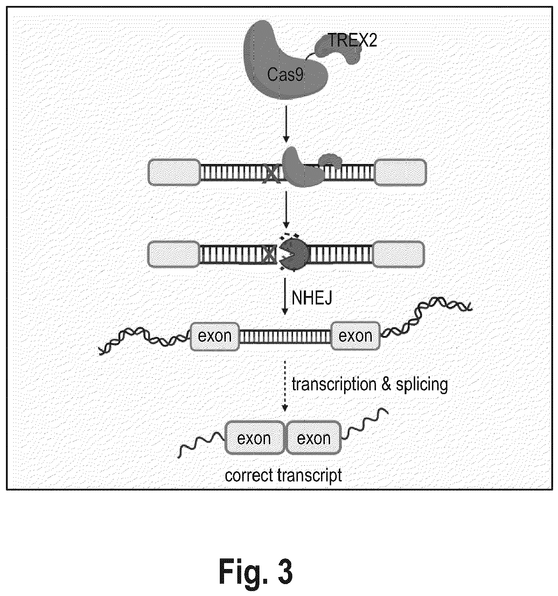

Another explicative example on how to target DIVs with a sgRNA complexed with the bioengineered ED-Cas9 variant according to the invention is shown in Fig. 3. On the other hand, ED-Cas9 is able to enhance the frequency occurrence of deletions, leading to the disruption of the sequence involved in determining missplicing. Upon the generation of a double-strand.

break mediated by SpCas9 paired to the same sgRNAs, the fusion partner TREX2 is able to further process the generated ends, leading to larger deletions. This translates in higher percentage of splicing rescue of the wild-type mRNA transcript. Moreover, ED-Cas9 enables flexibility in gRNA design as its disruption activity is also seen at a distance from the DIV.

### 2. ED-Cas9 applied to ABCA4

The inventors targeted a deep-intronic variant located in intron 36 of ABCA4 (c.5197-557G>T). Six gRNAs were used to target the mentioned DIV, both using SpCas9 or ED-Cas9, using the same minigene assay protocol. As controls, a wildtype minigene and a mutant c.5197-557G>T minigene were used. In short, a plasmid encoding for a fragment of *ABCA4* gene including the DIV is transfected in HEK293T cells alongside a plasmid encoding for either SpCas9 or ED-Cas9 (ED-Cas9 vector) and a given sgRNA.

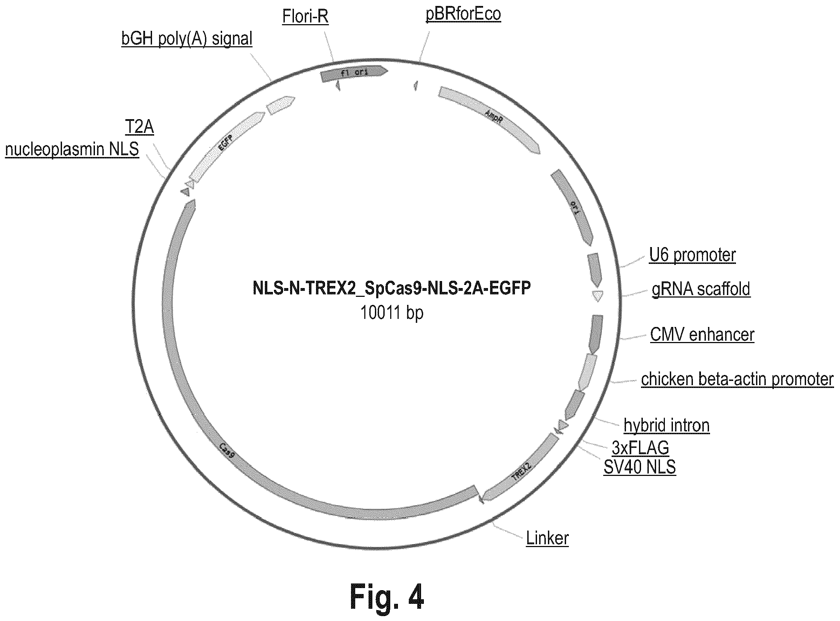

The map of the ED-SpCas9 vector is shown in detail in Fig. 4. ED-SpCas9 (enhanced deletion Cas9) is composed of (from N-terminus to C-terminus) the human exonuclease *TREX2* cDNA, a -GGGGS- linker and the *Streptococcus pyogenes* Cas9 (SpCas9) cDNA. A SV40 nuclear localization signal (NLS) is added to the N-terminus of TREX2 and a nucleoplasmin NLS is added to the C-terminus of SpCas9. The SpCas9 sequence can be replaced by any Cas9 orthologous sequence. The ED-SpCas9 protein can be also produced as a recombinant protein and used as such. In the shown in Fig. 4, ED-SpCas9 is also tagged with a T2A-EGFP marker. The promoter driving the transcription of ED-SpCas9 is the synthetic CAG promoter: CMV enhancer, chicken beta-actin promoter and a hybrid intron.

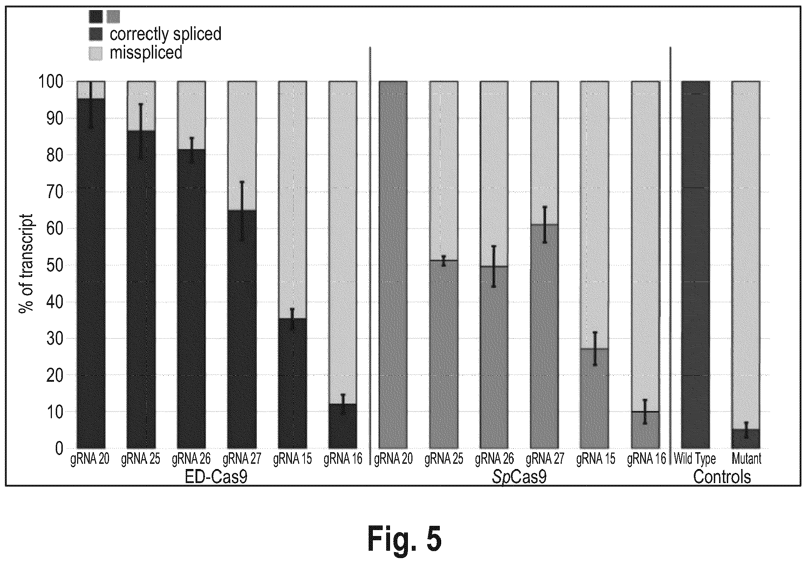

*ABCA4* mRNA is then extracted and analyzed to study the fraction of rescued, meaning correctly spliced transcript versus misspliced transcripts. As can be seen in Fig. 5 this experiment results in the retention of a 188-bp intronic sequence in the mature mRNA transcript which ultimately leads to nonsense-mediated mRNA decay. Therefore, this strategy allows to preliminary screen the rescue potential of a given strategy.

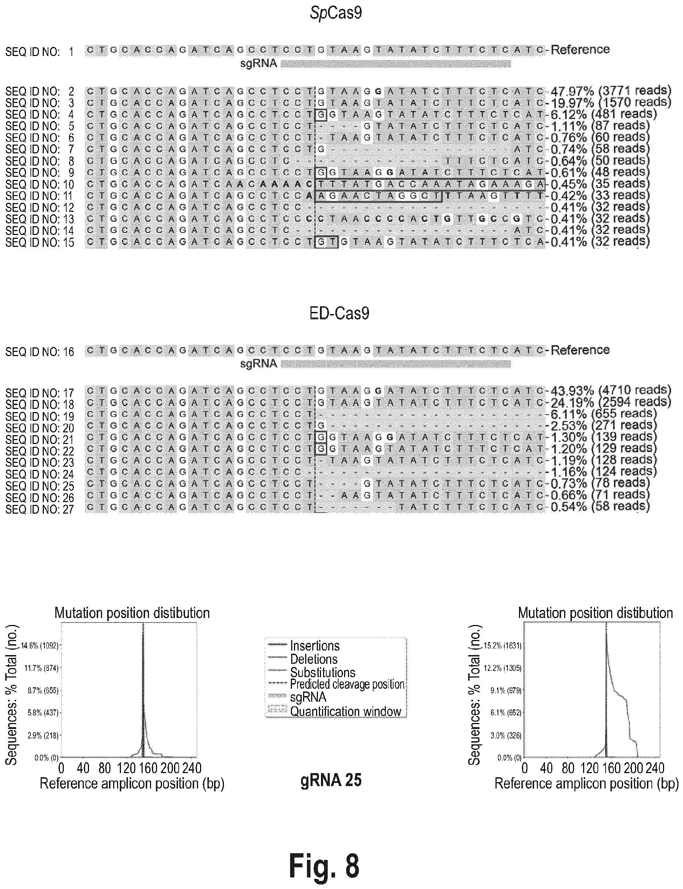

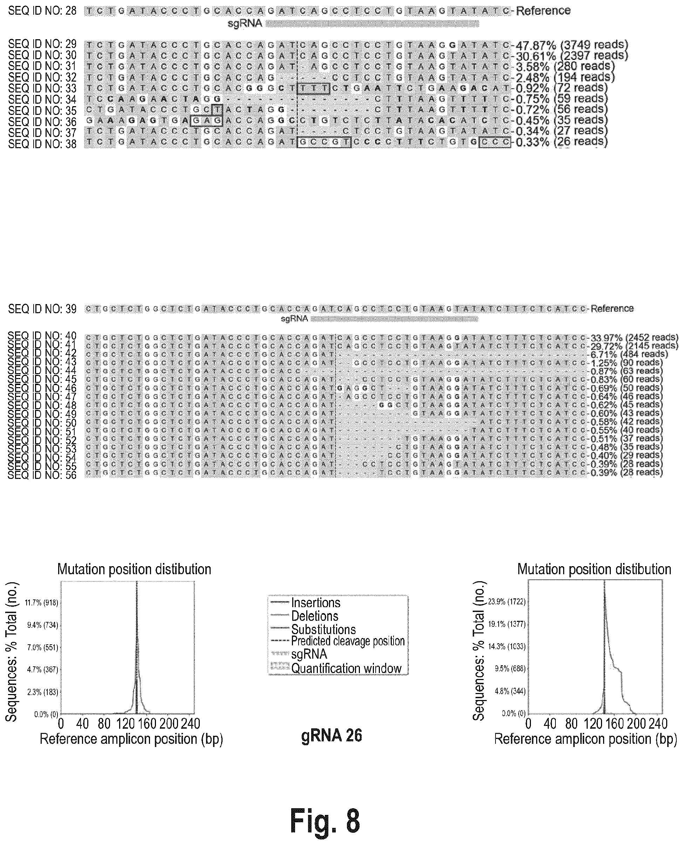

gRNA 20, gRNA 25, gRNA 26 and gRNA 27 induce single or double strand breaks upstream the deep-intronic variant, whereas gRNA 15 and gRNA 16 do downstream. gRNA 20, gRNA 25 and gRNA 26 also overlap with the DIV. In general, better rescue is observed when using gRNAs that are targeting upstream of the site of interest. This is due to the fact that TREX2 first processes the 3' extremity of the non-target strand, therefore most of the deletions are biased towards that side, as can be appreciated also in Fig. 8.

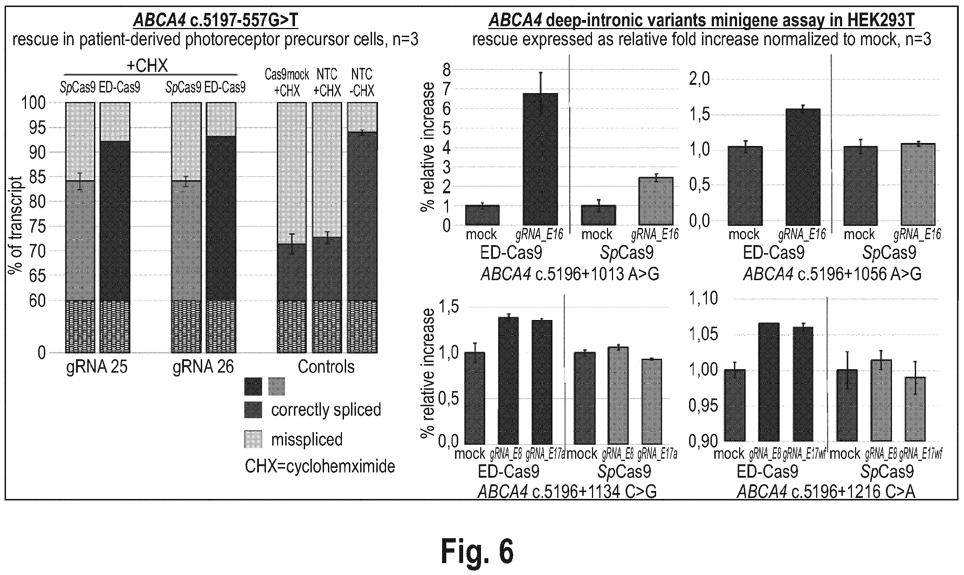

Two of the screened sgRNAs (gRNA25 and gRNA26) were then tested in patient-derived photoreceptor precursor cells showing appreciable improvement of the rescued transcript. Of note, the patient-derived cell line we obtained for c.5197-557G>T is heterozygous for the DIV. As can be seen on the graph below, untreated cells already show 71%±1 of correctly spliced *ABCA4* transcript. After the SpCas9 treatment the fraction of the correctly spliced increased to 85%, whereas upon ED-Cas9 treatment it is increased to 93% (about 50% more compared to SpCas9). Cycloheximide (CHX) has to be added to block the degradation of the misspliced transcript. CHX is a small molecule able to block the nonsense-mediated mRNA decay pathway. The results are shown in Fig. 6: A) Patient-derived induced pluripotent stem cells were differentiated in photoreceptor precursor cells to boost the expression of ABCA4. 30-day-old photoreceptor precursor cells were delivered with gRNA 25 and gRNA 26 complexed to ED-SpCas9 and SpCas9 for comparison. CHX (cyclohexamide) is added to block nonsense-mediated mRNA decay. The controls used are Cas9mock+CHX (no gRNA), untransfected cells + CHX (NTC+CHX) and untransfected cells without CHX (NTC-CHX). B) Four clustered DIVs were targeted using ED-SpCas9 and SpCas9 for comparison. The results are expressed as relative fold increase of the correctly spliced ABCA4 transcript normalized to mock.

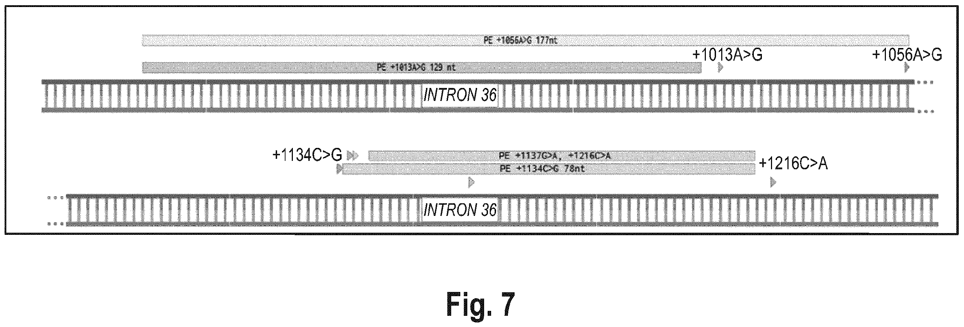

In addition, other clustered DIVs in *ABCA4* (intron 36) were targeted using a minigene assay protocol. *ABCA4* c.5196+1013A>G and *ABCA4* c.5196+1056A>G result in the retention of pseudo-exons sharing the same cryptic acceptor splicing site (129 bp and 177 bp pseudo-exons, respectively. Fig. 7). Similarly, *ABCA4* c.5196+1134C>G and *ABCA4* c.5196+1216 C>A determine the retention of a 78 bp and a 73 bp pseudo-exons, respectively, sharing the same cryptic donor splicing site (Fig. 7). To target these clustered DIVs, the inventors focused on assessing the potential of ED-SpCas9 in targeting common features deriving from the DIVs molecular mechanism, so as to use single gRNAs able to rescue different DIVs. After preliminary gRNA screening, gRNA E16 was selected as most effective in rescuing the splicing of *ABCA4* c.5196+1013A>G and *ABCA4* c.5196+1056A>G. Whereas, gRNA E8 was chosen for the rescue of *ABCA4* c.5196+1134C>G and *ABCA4* c.5196+1216 C>A (Fig. 6B). gRNA_E17a and gRNAE17_wt partially overlap with the *ABCA4* c.5196+1134 position, therefore a single gRNA cannot be used to simultaneously target *ABCA4* c.5196+1013A>G and *ABCA4* c.5196+1056A>G. Nonetheless, this gRNA was selected for further experiment in patient-derived photoreceptor precursor cells because of other DIVs present in the cluster which cannot be assessed by minigene assay. Overall, the results of the minigene assay show considerable higher rescue mediated by ED-SpCas9 compared to SpCas9.

By means of high-throughput sequencing, the inventors also performed mutational profiling of the resulting editing at the genomic level in patient-derived photoreceptor precursor cells for SpCas9 and ED-SpCas9 coupled with gRNA 25 and gRNA 26. The results are shown in Fig. 8. The top part for each gRNA section reports the composition of the mutational profiles. The graphs in the bottom parts show the % and type of mutational repairs plotted against the reference amplicon position.

As clearly shown in Figure 8 ED-SpCas9 is able to consistently achieve larger deletion when compared to SpCas9. Furthermore, the cut efficiency of SpCas9 and ED-SpCas9 is comparable. Of note, the cell line used is heterozygous for the targeted deep-intronic variant (c.5197-557G>T), gRNA 25 and gRNA 26 overlap with the deep-intronic mutation, therefore they can only effectively target the mutant allele.

### 3. ED-SpCas9 applied to RHO

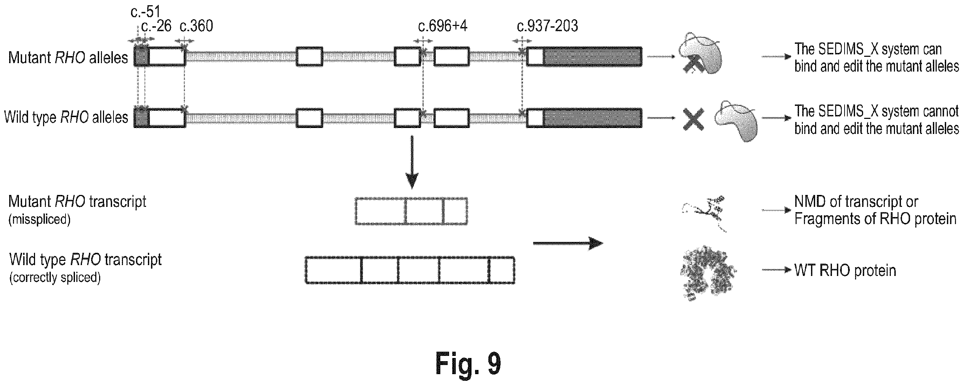

The inventors apply a novel strategy, which they named 'Selective disruption of the mutant allele through targeting common SNPs by Cas9-exo' (SEDIMS-X), which combines the superior efficacy of single targets with the broader applicability of the SNP-based mutation independent approach (Fig. 9). To overcome the constraints of targeting just SNPs within or in very close vicinity with functionally relevant sequences (i.e. exons, transcriptional and translational regulation), the inventors employ Cas9 fused to an exonuclease (Cas9-exo) which expands the size of the generated indels and was shown to perform superior in the functional elimination of *ABCA4* pseudoexons.

Considering *in vivo* application and clinical translation of the SEDIMS-X approach, in addition to standard *Streptococcus pyogenes* Cas9-exo (SpCas9) as benchmark two small Cas9 orthologous can be used which are active in mammalian cells and which can be packed in AAV particles for the approach: *Neisseria meningitides* Cas9 (Nme2Cas9) and *Staphylococcus auricularis* Cas9 (SauriCas9). Moreover, due to the permissive protospacer adjacent motif (PAM) requirements of the selected Cas9 orthologues (N₄CC for Nme2Cas9, NNGG for SauriCas9 and NGG for SpCas9), higher flexibility in targeting defined DNA sequences is granted.

The basic principle of the SEDIMS-X approach adapted to the common SNPs in RHO is outlined in Fig. 9. Single SNPs in *cis* with the mutation are targeted by allele-specific gRNAs and mutant alleles efficiently disrupted by a Cas9-exonuclease fusion enzyme.

Feasibility of the SEDIMS-X approach relies on the presence of common polymorphisms in the vicinity of the coding sequences or crucial regulatory sequences. The inventors queried the Genome Aggregation Database (gnomAD) to identify and to retrieve the frequency of common SNPs in *RHO*. Relevant SNPs are reported in (Table 1), and assessed the number of potential allele-specific gRNAs based on PAM requirements (59 gRNAs). The analysis of the inventors show the presence of a considerable number of common SNPs in the target gene potentially suited for the SEDIMS-X approach.

**Table 1: Population frequencies of common SNPs in the rhodopsin (RHO) gene (Source: gnomAD). ^{a} - Reference Sequences: NM_000539.3, ^{b}-c.696+4C>T variant also in linkage disequilibrium with the common c.68C>A;p.Pro23His mutation, ^{c}-potential allele-specific gRNAs based on PAM motif requirements.**

| SNP | | gRNAs^{c} | | |
|---|---|---|---|---|
| Position^{a} | Minor allele frequency in gnomAD (in heterozygosity) | Nme2Cas9 | SauriCas9 | SpCas9 |
| c.360C>T | 0.003 | 5 | 10 | 10 |
| c.696+4C>T^{b} | 0.08 | 3 | 9 | 10 |
| c.937-23G>A | 0.05 | 5 | 3 | 4 |

The inventors further analyzed the frequency of four of these SNPs in 36 adRP patients carrying *RHO* class-II mutations, and found a cumulative frequency of 18% of subjects being heterozygous for one or multiple of these common SNPs (Table 2). Notably, the minor c.696+4C>T allele has been shown to be in linkage disequilibrium with the c.68C>A;p.Pro23His mutation which is the most common *RHO* mutation in patients form the US (25). Remarkably relevant for this approach, targeting of a C-terminus amino acid (P347S) results in lower accumulation of misfolded RHO and reduces the expression of the mutant protein in mouse photoreceptors. This proves that c.937-23 position is still a valid SNP for this approach.

**Table 2: Patients being heterozygous for selected common SNPs among a cohort of 36 adRP patients carrying class II-RHO mutations. ^{a} - polymorphism c.937-23G>A not tested in this patient series.**

| | | |
|---|---|---|
| Polymorphism^{a} | c.360C/T | c.696+4C>T |
| Heterozygosity | 3% | 15% |
| Cumulative frequency | 18% | |

An iPSC cell line from a patient with the mutation c.644C>T;p.Pro215Leu and heterozygous for the SNP c.360C/T is generated in-house, and additional patients with suitable genotypes (class II RHO mutation, heterozygous for one of the selected SNPs) are shortlisted for a skin biopsy upon their next clinical appointment.

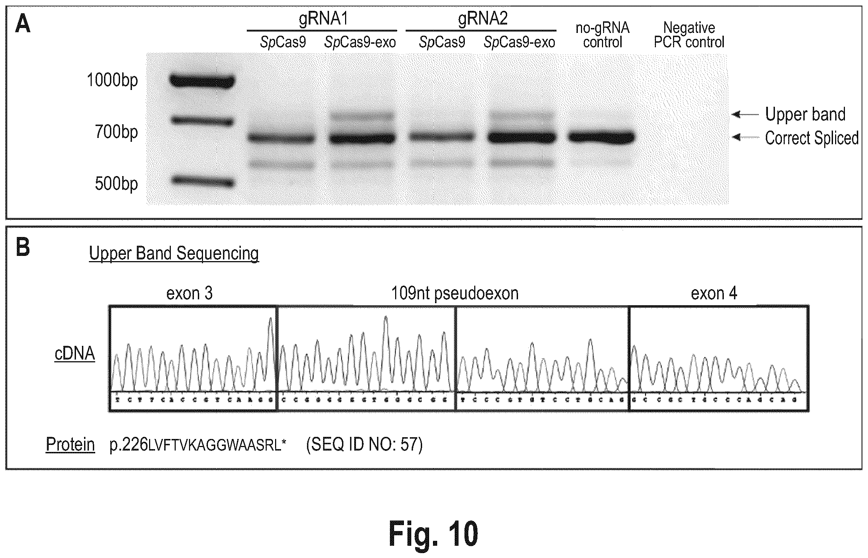

To prove the effectiveness and superiority of the SEDIMS-X approach applying a Cas9-exo fusion enzyme in a pilot experiment, the inventors generated a minigene construct (pSPL3-RHO) with the full length *RHO* gene (including introns) and targeted nucleotide position c.696+4 with two different gRNAs. Transcript analysis of HEK293T cells co-transfected with pSPL3-RHO and SpCas9-exo/U6.gRNA or SpCas9/U6.gRNA expression constructs showed an additional misspliced RT-PCR product (Fig. 10, upper pane) in cells expressing SpCas9-exo. Sequencing revealed disruption of the exon 3 - intron 3 boundary and retention of most of intron 3 resulting in frame-shift and premature stop codon (Fig. 10).

As a functional read-out for our SEDIMS-X approach, the inventors established a recently described flow cytometry-based assay to determine surface expression of rhodopsin which is strongly impaired in class II *RHO* mutants. To mimic the effect of allele-specific editing in heterozygous state, HEK293T cells were transfected with different ratios of wild-type and mutant RHO expression constructs, and - applying an anti-RHO antibody on non-permeabilized cells - assayed for plasma membrane-resident RHO by flow cytometry. For both class II mutants tested (p.P53R, p.R135W), the inventors observed a reliable increase in surface expression at mutant-to-wildtype ratios (25:75 and 15:85) which are in a realistic range that can be achieved by this approach (Fig. 11).

### 4. ED-SpCas9 applied to USH2A

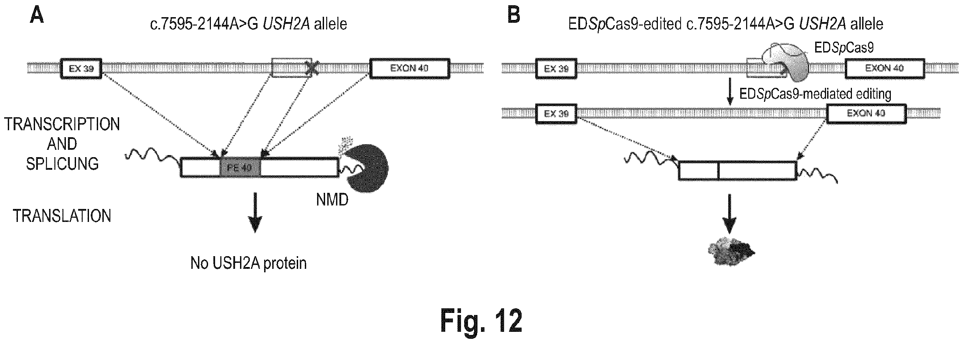

*USH2A*:c.7595-2144A>G is a common deep intronic variant (DIV) that activates a cryptic splice site in intron 40, resulting in pseudoexon retention (PE40) in the mature mRNA (Fig. 12). By means of Cas9-mediated genome editing, such cryptic splice sites can be perturbed, hereby rescuing to normal splicing. The inventors have bioengineered a novel Enhanced-Deletion Cas9 (EDSpCas9) that can destruct c.7595-2144A>G using single gRNAs by promoting deletions at c.7595-2144A>G, resulting in disruption of the cryptic splice site and rescuing of normal splicing (Fig. 12).

This approach demonstrates the efficacy of the novel ED-SpCas9 molecule to rescue the *USH2A*:c.7595-2144A>G DIV. The inventors select and assess sgRNAs for their rescue potential using minigene assays in HEK293T cells. Selected sgRNAs are evaluated in homo- and compound heterozygous *USH2A*:c.7595-2144A>G patient-derived fibroblast cell lines. The gDNA editing spectrum, mRNA splicing rescue and restored protein expression are thoroughly characterized. Based on the results obtained, the inventors deal with the functional characterization of validated sgRNAs in patient-derived fibroblasts and photoreceptor precursor cells (PPCs) by means of a ciliogenesis assay.

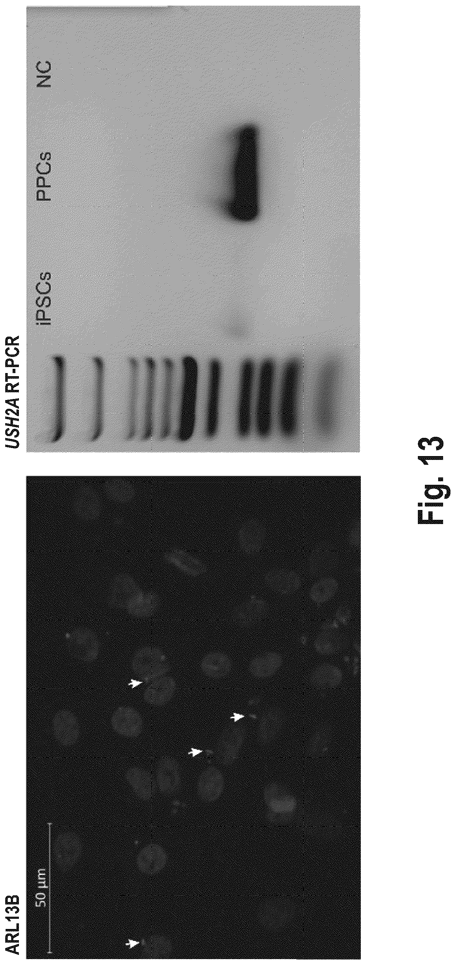

The inventors performed antibody staining with the cilia marker ARL13B on 30-day-differentiated PPCs. As shown in the Fig. 13, most of the PPCs show clear presence of cilia elongation. Furthermore, by means of RT-PCR, the inventors investigated the expression of *USH2A* in iPSCs and 30-day differentiated PPCs. As shown in Fig. 13, PPCs express *USH2A,*

## Claims

1. A gene editing system for editing genomic DNA to modulate splicing comprising:
- a nucleic acid encoding an RNA-guided endonuclease molecule or fragment thereof fused to a molecule comprising exonuclease activity (ED-RgE molecule), and
- one single guide RNA (sgRNA) molecule,
wherein said ED-RgE molecule and said sgRNA molecule are configured to associate with genomic DNA comprising a splicing site and to cause a deletion in said splicing site resulting in a modulation of splicing.

2. The gene editing system of claim 1, wherein said RNA-guided endonuclease molecule is a Cas9 molecule, preferably said Cas9 is selected from the group consisting of: *Streptococcus pyogenes* Cas9 (SpCas9), *Neisseria meningitides* Cas9 (Nme2Cas9), and *Staphylococcus auricularis* Cas9 (SauriCas9).

3. The gene editing system of any of the preceding claims, wherein said modulation of splicing is a splicing rescue.

4. The gene editing system of any of the preceding claims, wherein said modulation of splicing is a missplicing.

5. The gene editing system of any of the preceding claims, wherein said splicing site is selected from the group consisting of: intronic variant/mutation, deep intronic variant/mutation, single nucleotide polymorphism (SNP), intronic and exonic SNP, donor splice site, acceptor splice site, branch point, slicing enhancer, splicing silencer.

6. The gene editing system of any of the preceding claims, wherein said molecule comprising exonuclease activity comprises TREX2 exonuclease activity.

7. The gene editing system of claim 6, wherein said ED-RgE molecule comprises a human TREX2 fused to a SpCas9 molecule (ED-Cas9 molecule).

8. The gene editing system of claim 7, wherein the nucleic acid encoding said ED-Cas9 molecule comprises a nucleotide sequence which is at least 85% identical to the nucleotide sequence of SEQ ID NO: 58, and, preferably, wherein said ED-Cas9 molecule comprises an amino acid sequence which is at least 85% identical to the amino acid sequence of SEQ ID NO: 59, and, preferably, wherein the nucleic acid encoding said ED-Cas9 molecule comprises a nucleotide sequence which is at least 85% identical to the nucleotide sequence of SEQ ID NO: 60, and, preferably, wherein said ED-Cas9 molecule comprises an amino acid sequence which is at least 85% identical to the amino acid sequence of SEQ ID NO: 61.

9. The gene editing system of any of the preceding claims, wherein said genomic DNA encodes a gene associated with an inherited disorder, preferably a retinal disease, further preferably an inherited retinal dystrophy (IRD), further preferably said gene is selected from the group consisting of: *ABCA4, RHO, USH2A, GUCY2D, RP1L1,* and *PROM1,* highly preferably of human origin.

10. The gene editing system of any of the preceding claims wherein said splicing site is selected from the group consisting of:
- *ABCA4*: c.5197-557G>T, c.5196+1013A>G, c.5196+1056A>G, c.5196+1134C>G, c.5196+1137G>A, c.5196+1216 C>A; and/or
- *RHO*: c.360C>T, c.696+4C>T, c.68C>A, c.937-23G>A, c.360C>T; and/or
- *USH2A:* c.7595-2144A>G.

11. A polynucleotide or vector encoding the gene editing system of any of claims 1-10.

12. A gene editing system for editing genomic DNA to modulate splicing comprising:
- a fusion protein comprising an RNA-guided endonuclease molecule or fragment thereof fused to a molecule comprising exonuclease activity (ED-RgE molecule), and
- one single guide RNA (sgRNA molecule),
wherein said ED-RgE molecule and said sgRNA molecule are configured to associate with genomic DNA comprising a splicing site and to cause a deletion in said splicing site resulting in a modulation of splicing.

13. A pharmaceutical composition comprising the gene editing system of any of claims 1-11 or 13, or the polynucleotide or vector of claim 12.

14. A method of editing genomic DNA in a cell to modulate splicing, the method comprising
- contacting the cell with
- a nucleic acid encoding an RNA-guided endonuclease molecule or fragment thereof fused to a molecule comprising exonuclease activity (ED-RgE molecule)
or/and
an ED-RgE molecule, and
- one single guide RNA (sgRNA molecule),
wherein said ED-RgE molecule and said sgRNA molecule are configured to associate with genomic DNA comprising a splicing site and to cause a deletion in said splicing site resulting in a modulation of splicing,
- incubating the cell under conditions allowing
- said ED-RgE molecule to by synthesized, if applicable;
- said ED-RgE molecule and said sgRNA molecule to associate with said genomic DNA, and
- to cause a deletion in said splicing site resulting in the modulation of splicing.

15. Use of a nucleic acid encoding an RNA-guided endonuclease molecule or fragment thereof fused to a molecule comprising exonuclease activity (ED-RgE molecule), or the ED-RgE molecule for the modulation of splicing.
